(19) **Europäisches Patentamt**

**European Patent Office**

**Office européen des brevets**

(11) **EP 1 317 907 A1**

(12) **EUROPÄISCHE PATENTANMELDUNG**

(43) Veröffentlichungstag:
**11.06.2003 Patentblatt 2003/24**

(51) Int Cl.⁷: **A61B 17/22, A61B 6/12**

(21) Anmeldenummer: **02024041.2**

(22) Anmeldetag: **28.10.2002**

(84) Benannte Vertragsstaaten:
**AT BE BG CH CY CZ DE DK EE ES FI FR GB GR
IE IT LI LU MC NL PT SE SK TR**
Benannte Erstreckungsstaaten:
**AL LT LV MK RO SI**

(30) Priorität: **10.12.2001 DE 10160532**

(71) Anmelder: **Dornier Medtech GmbH
82234 Wessling (DE)**

(72) Erfinder: **Neumann, Jürgen
82239 Alling (DE)**

(74) Vertreter: **Knauer, Reinhard et al
Grünecker, Kinkeldey
Stockmair & Schwanhäusser
Anwaltssozietät
Maximilianstrasse 58
80538 München (DE)**

(54) **Verfahren und Vorrichtung zur dreidimensionalen Ortung eines Konkrements**

(57) Verfahren zur Ortung eines Konkrements (3), beispielsweise eines Nieren- oder Gallensteins im Körper (1) eines Lebewesens zur Positionierung einer Therapievorrichtung wie etwa eines Lithotripsiegeräts, bei dem eine im wesentlichen senkrechte Projektionsabbildung des Konkrements mittels einer Strahlungsquelle (5) auf einem Abbildungsfenster (6) erzeugt wird, der Körper in der Körperebene relativ zur Strahlungsquelle (5) verfahren, mit einer erneuten Projektionsabbildung die Projektion d' des Verfahrwegs erfaßt und die vertikale z-Position des Konkrements als

$z = z' \cdot \dfrac{d}{d'}$ berechnet wird, wobei z' der feste Abstand zwischen Strahlungsquelle (5) und Abbildungsfenster (6) ist.

Strahlungsquelle

## Fig. 5

z

Z'

Steinlage (Objektebene)

3

Eingangsfenster (Bildebene)

d

d'

EP 1 317 907 A1

**Beschreibung**

Technisches Gebiet

Die Erfindung betrifft die Ortung von Konkrementen wie etwa Gallen- oder Nierensteinen im Körper von Lebewesen für die Positionierung von Therapiesystemen, insbesondere von Therapiesystemen zur Stoßwellentherapie.

Stand der Technik

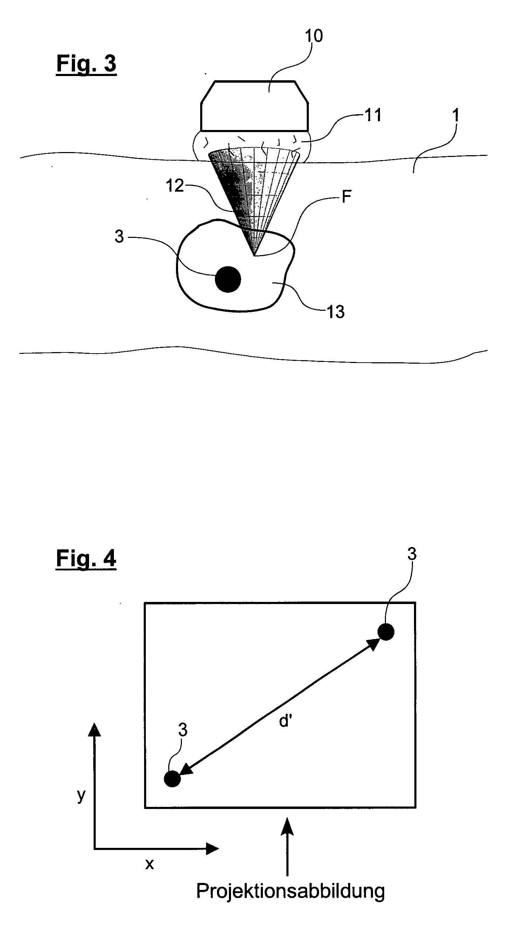

In den vergangenen 15 bis 20 Jahren hat sich die Stoßwellentherapie oder Lithotripsie als nicht-invasive Therapieform zur Behandlung von Patienten mit Nieren-, Gallen- oder Blasensteinen etabliert. Das Prinzip der Stoßwellentherapie ist schematisch in Fig. 3 illustriert. Eine Stoßwellenquelle erzeugt mittels eines Transducers Schallwellen, die über ein mit gel- oder wasserartigem Material gefülltes Einkoppelkissen 11 auf den Körper 1 des zu behandelnden Patienten übertragen werden. Das Stoßwellenfeld 12 wird auf einen Brennpunkt F fokussiert. Um das Konkrement (den Stein 3) in einem Organ 13 durch die Stoßwellen in genügend kleine Bruchstücke zertrümmern zu können, ist es erforderlich, daß der Fokus F des Stoßwellenfelds 12 das Konkrement 3 trifft. Es ist daher eine genaue Ortung des Steins 3 zur nachfolgenden Positionierung der Stoßwellenquelle 10 erforderlich.

Die Ortung des Konkrements 3 erfolgt häufig mittels Röntgenstrahlung, wobei eine senkrecht auf den Körper auftreffende und diesen durchleuchtende Röntgenstrahlung (ap-Projektion) mit einer schräg auf denselben Oberflächenbereich des Körpers auftreffenden und diesen durchleuchtenden Röntgenstrahlung (cc-Projektion) kombiniert wird. Mit der senkrechten Röntgenstrahlung kann direkt die Horizontalposition (X-Y-Ebene) des Konkrements bestimmt werden. Daraufhin wird die Röntgenquelle um einen festgelegten Winkel, beispielsweise 30° zur Vertikalen, d.h. der ursprünglichen Projektionsrichtung (ap) gedreht, wodurch eine Vertikalposition des Konkrements ermittelt werden kann.

Dann wird - unter schräg einfallender Röntgeneinstrahlung und Beibehaltung der horizontalen Position - der zu behandelnde Patient mittels eines Behandlungstisches solange in Vertikalrichtung verfahren, bis der zu beseitigende Stein in der richtigen vertikalen Position (z-Richtung) im Fokus der Stoßwellenquelle liegt. Eine derartige Ortungsvorrichtung ist in der Offenlegungsschrift DE 19513400 A1 beschrieben.

Das beschriebene Ortungsverfahren weist jedoch den Nachteil auf, daß das Schrägstellen der oftmals großen und schweren Röntgengeräte mühsam und bei bestimmten Konstruktionen gar nicht möglich ist. Bei bestimmten Anwendungen, z.B. Urotischen, ist die Einstrahlrichtung der Röntgenquelle auf den Patienten fest, in der Regel AP.

Der vorliegenden Erfindung liegt die Aufgabe zugrunde, die im Stand der Technik vorhandenen Nachteile zu überwinden und ein Ortungsverfahren und eine Ortungsvorrichtung für Konkremente mittels einer Bildgebungsvorrichtung vorzuschlagen, bei der eine Bestrahlung des Körpers aus verschiedenen Raumrichtungen nicht erforderlich ist.

Zusammenfassung der Erfindung

Gelöst wird die Aufgabe durch ein Verfahren zur dreidimensionalen Ortung eines Konkrements im Körper eines Lebewesens zur Positionierung einer Therapievorrichtung, wobei eine im wesentlichen senkrechte Projektionsabbildung des Konkrements mittels einer bildgebenden Strahlungsquelle erzeugt, die Abbildungskoordinaten des Konkrements bestimmt, der Körper relativ zur Strahlungsquelle horizontal verfahren und der Verfahrweg d erfaßt wird, die Projektion d' des Verfahrwegs erfaßt und aus den gewonnenen Größen d, d' und dem Abstand z' der Strahlungsquelle von der Bildebene der bildgebenden Vorrichtung die vertikale Position des Konkrements ermittelt wird.

Aus der Auslegeschrift DE 1029122 ist eine Vorrichtung und ein Messverfahren zur Bestimmung von Lage und Größe von Fremdobjekten in Durchleuchtungskörpern wie etwa Patienten bekannt, bei denen die horizontale und vertikale Position eines Objektes im Körper mittels zweier Markierungen B1, B2 auf dem Leuchtschirm des Röntgengeräts ermittelt wird. Der Arbeitsweise der Vorrichtung liegt der Satz vom Verhältnis der Seiten in ähnlichen Dreiecken zu Grunde. Dabei wird die vertikale Position eines Konkrements ermittelt, indem die Abmessungen ähnlicher Dreiecke miteinander in ein Verhältnis gesetzt werden.

Durch das erfindungsgemäße Verfahren wird es möglich, die vollständige dreidimensionale Position eines Konkrements im Körper lediglich durch zwei senkrechte Projektionsabbildungen etwa mittels Röntgenstrahlen zu bestimmen. Es ist lediglich erforderlich, daß der Körper relativ zur Strahlungsquelle um einen Verfahrweg d horizontal - in Körperebene - verfahren wird. Ein Schrägstellen der Strahlungsquelle ist somit nicht erforderlich.

Die Relativbewegung zwischen Körper und Strahlungsquelle kann durch einen horizontal beweglichen Behandlungstisch oder eine horizontal bewegliche Bildgebungsvorrichtung realisiert werden.

Vorzugsweise wird erfindungsgemäß der vertikale Abstand z des Konkrements von der Strahlungsquelle bestimmt als $z = z' \cdot \frac{d}{d'}$. Die Positionsgenauigkeit nimmt mit der Größe des Verfahrwegs d zu. Vorzugsweise wird daher zur vertikalen Positionsbestimmung des Konkrements ein zur Verfügung stehender Abbildungsbereich der Bildgebungsvorrichtung maximal ausgenutzt, beispielsweise die Diagonale eines rechteckigen Abbildungsbereichs oder der Durchmesser eines runden Abbildungsbereichs.

Die verwendete bildgebende Strahlung kann vorzugsweise Röntgenstrahlung sein. Jede andere Strahlung, die eine senkrechte Projektionsabbildung des Körpers liefert ist jedoch ebenfalls anwendbar.

Die Erfindung schlägt außerdem eine Vorrichtung zur Ortung eines Konkrements im Körper gemäß Anspruch 8 vor.

Die untergeordneten Ansprüche beschreiben weitere bevorzugte Ausgestaltungen der Erfindung.

Kurze Beschreibung der Zeichnungen

Weitere Aufgaben, Vorteile und Merkmale der vorliegenden Erfindung werden anhand der Beschreibung eines Ausführungsbeispiels unter Bezugnahme auf die beiliegenden Zeichnungen deutlich, in denen:

Fig. 1    eine schematische Seitenansicht einer erfindungsgemäßen Ortungsvorrichtung ist;

Fig. 2    eine Vorderansicht der erfindungsgemäßen Ortungsvorrichtung ist;

Fig. 3    schematisch die Stoßwellentherapie eines Konkrements illustriert;

Fig. 4    die Projektion d' eines Verfahrwegs d auf der Bildebene zeigt;

Fig. 5    eine schematische Darstellung zur Erläuterung der Funktionsweise des erfindungsgemäßen Ortungsverfahrens ist; und

Fig. 6    die Verfahrensschritte des erfindungsgemäßen Ortungsverfahrens zeigt.

Beschreibung eines bevorzugten Ausführungsbeispiels

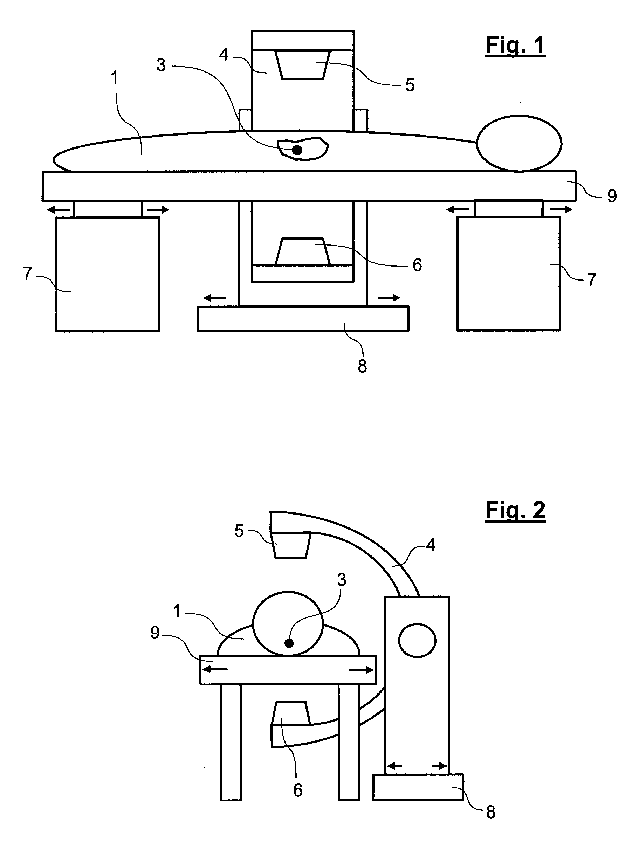

Fig. 1 zeigt in Seitenansicht eine erfindungsgemäße Vorrichtung zur dreidimensionalen Ortung eines Konkrements 3 im Körper eines Lebewesens, beispielsweise eines Patienten 1. Der Patient liegt auf einem Behandlungstisch 9, der beispielsweise in Körperlängsrichtung und/oder in Körperquerrichtung (siehe Vorderansicht in Fig. 2) bewegbar ist. Ein Röntgengerät 4 (C-Bogen) als Bildgebungssystem ist auf einem Sockel 8 montiert, der eine Bewegung des Röntgengeräts in Patientenlängsrichtung und/oder -querrichtung erlaubt. Erfindungsgemäß ist es ausreichend, wenn entweder der Behandlungstisch 9 oder das Röntgengerät 4 horizontal bewegbar sind.

Das Bildgebungssystem 4 weist eine Strahlungsquelle 5 auf, die senkrecht durchleuchtende Röntgenstrahlung (ap-Projektion) auf den Körper 1 aussendet und im Bildfenster 6 eine Projektionsabbildung des durchleuchteten Körperabschnitts erzeugt. Der Abstand z' zwischen Strahlungsquelle 5 und Bildfenster bzw. Bildebene 6 bleibt während der Ortungsprozedur immer konstant. Außerdem werden ausschließlich ap-Projektionsaufnahmen erstellt.

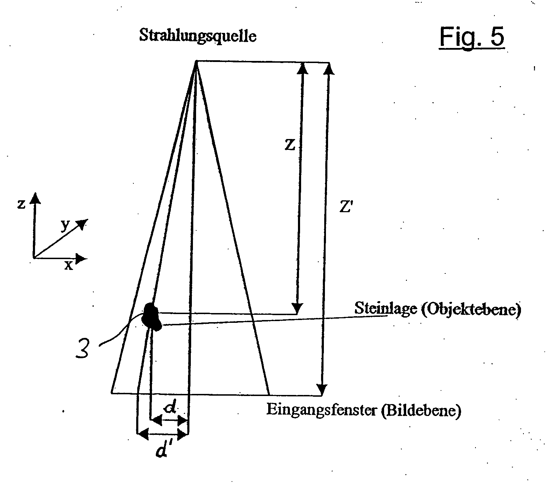

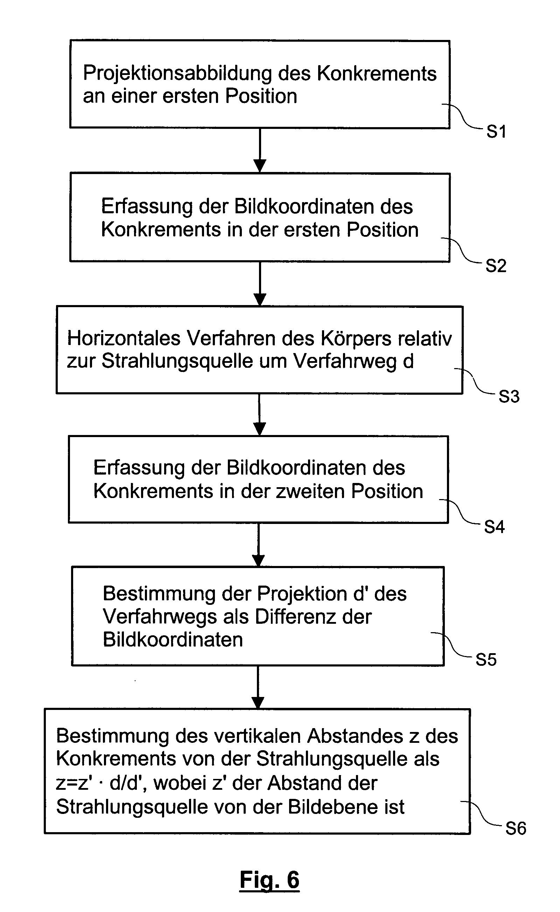

Das erfindungsgemäße Ortungsverfahren ist schematisch in Fig. 6 illustriert. Zunächst wird der Patient relativ zur Bildgebungseinrichtung so positioniert, daß sich das Konkrement 3 an einem Randbereich des Abbildungsfensters der Bildgebungseinrichtung befindet, beispielsweise in der rechten oberen Ecke des in Fig. 4 illustrierten Bildbereichs. Dann wird in Verfahrensschritt S1 (Fig. 6) eine Projektionsabbildung des Konkrements in dieser Position durchgeführt und die Bildkoordinaten des Konkrements 3 erfaßt (Schritt S2). Die Ortung des Konkrements in der Körperebene (Horizontalebene, X-Y-Ebene) ist damit schon erreicht. Zur vertikalen Ortung wird im anschließenden Verfahrensschritt S3 der Körper relativ zur Strahlungsquelle um den Verfahrweg d horizontal verfahren. Dies kann durch Bewegung des Behandlungstisch in X- und/oder Y-Richtung oder des Röntgengeräts in X- und/oder Y-Richtung erfolgen. Der tatsächliche horizontale Verfahreweg d des Körpers relativ zur Bildgebungseinrichtung wird dabei durch eine (nicht dargestellte) Meßeinrichtung erfaßt.

In den Schritten S4 und S5 wird eine Projektionsabbildung des Konkrements 3 an der neuen Position erzeugt und die Projektion d' des Verfahrwegs als Differenz der Bildkoordinaten des Konkrements an den beiden Abbildungspositionen bestimmt. Bei der Wahl des Verfahrwegs d ist darauf zu achten, daß das Konkrement innerhalb des Bildbereichs der Bildgebungsvorrichtung bleibt. Da die Meßgenauigkeit mit einer Zunahme des Verfahrwegs d zunimmt, ist eine maximale Ausnutzung des Abbildungsbereichs wie in Fig. 4 gezeigt vorteilhaft. Dort ist die Projektion d' des Verfahrwegs von einer Anfangsposition rechts oben zu einer Endposition links unten gezeigt.

Im folgenden Verfahrensschritt S6 wird der vertikale Abstand z des Konkrements von der Strahlungsquelle 5 als $z = z' \cdot \frac{d}{d'}$ bestimmt, wobei z' der Abstand der Strahlungsquelle 5 von der Bildebene 6 ist. Somit sind alle drei Raumkoordinaten X, Y und Z des Konkrements bekannt, so daß der Fokus F der Stoßwellenquelle 10 (siehe Fig. 3) in Übereinstimmung mit der räumlichen Position des Steins 3 gebracht werden kann.

Die Bestimmung der Vertikalkoordinate des Steins wird im folgenden anhand von Fig. 5 erläutert. Aus dem Strahlensatz ergibt sich, daß der vertikale Abstand z des Steins von der Strahlungsquelle zum vertikalen Abstand z' der Bildebene zur Strahlungsquelle dem Verhältnis von tatsächlichem Verfahrweg d zur Projektion d' des Verfahrwegs entspricht. Daraus ergibt sich:

$$z = z' \cdot \frac{d}{d'}.$$

Der Abstand von der Strahlungsquelle und damit die vertikale Position des Konkrements läßt sich also aus den Größen Verfahrweg d, Projektion des Verfahrwegs d' und Abstand zwischen Strahlungsquelle und Bildebene z' bestimmen. Die Größe z' ist konstant und die Größen d und d' lassen sich auf einfache Weise mittels zweiter Vertikalprojektionsabbildungen ermitteln. Mit der durch die Projektionsabbildung ermittelten X- und Y-Koordinaten des Konkrements erhält man so auf einfache Art und Weise die räumliche Position (x, y, z) des zu behandelnden Konkrements im Körper.

Bezugszeichenliste

| | |
|---|---|
| 1 | Körper |
| 3 | Konkrement |
| 4 | Projektionsabbildungsvorrichtung / Bildgebungsvorrichtung |
| 5 | Strahlungsquelle |
| 6 | Abbildungsfenster |
| 7 | angetriebener Behandlungstischfuß |
| 8 | Bewegungseinrichtung für Abbildungsvorrichtung |
| 9 | Behandlungstisch |
| 10 | Stoßwellenquelle |
| 11 | Einkoppelkissen |
| 12 | Stoßwellenfeld |
| 13 | Körperorgan |

**Patentansprüche**

1. Verfahren zur dreidimensionalen Ortung eines Konkrements (3) in einem Körper (1) eines Lebewesens zur Positionierung einer Therapievorrichtung, aufweisend die Schritte:

   - Erzeugen einer senkrechten Projektionsabbildung des Konkrements (3) mittels einer bildgebenden Strahlungsquelle,

   - Bestimmen der horizontalen Position des Konkrements (3) mittels der Projektionsabbildung,

   - Horizontales Verfahren des Körpers (1) relativ zur Strahlungsquelle (5) um einen Verfahrweg d,

   - Erfassen der Projektion d' des Verfahrwegs,

   - Bestimmen der vertikalen Position des Konkrements (3) aus den Größen Verfahrweg d, Projektion d' des Verfahrwegs und Abstand z' der Strahlungsquelle (5) von der Bildebene (6) der Projektionsabbildung.

2. Verfahren nach Anspruch 1, wobei der Abstand z des Konkrements (3) von der Position der Strahlungsquelle (5) bestimmt wird als $z = z' \cdot \frac{d}{d'}$.

3. Verfahren nach Anspruch 1 oder 2, wobei die Strahlungsquelle (5) eine Röntgenquelle ist.

4. Verfahren nach Anspruch 1, 2 oder 3, wobei der Körper (1) mittels eines beweglichen Behandlungstisches (9) relativ zur Strahlungsquelle (5) bewegt wird.

5. Verfahren nach Anspruch 1, 2 oder 3, wobei die Strahlungsquelle (5) fest mit dem Abbildungsfenster der Projektionseinrichtung verbunden ist und relativ zum Körper (1) bewegbar ist.

6. Verfahren nach einem der Ansprüche 1 bis 5, wobei die Relativbewegung so ausgeführt wird, daß ein zur Verfügung stehender Abbildungsbereich der Projektionsabbildung maximal ausgenutzt wird.

7. Verfahren nach Anspruch 6, wobei der Verfahrweg diagonal in einem rechteckigen Bildbereich der Projektionsabbildung ist.

8. Vorrichtung zur Ortung eines Konkrements im Körper (1) eines Lebewesens zur Positionierung einer Therapievorrichtung, aufweisend:

   - eine Bildgebungseinrichtung (4) mit einer Strahlungsquelle (5) und einem Abbildungsfenster (6), die voneinander einen festen Abstand z' aufweisen, zur Erzeugung einer senkrechten Projektionsabbildung des Konkrements (3) und Bestimmungen der horizontalen Position des Konkrements,

   - eine Einrichtung zum horizontalen Verfahren des Körpers (1) relativ zur Strahlungsquelle (5) und zur Erfassung des Verfahrwegs d,

   - eine Einrichtung zur Erfassung der Projektion d' des Verfahrwegs im Abbildungsfenster (6), und

   - eine Einrichtung zur Bestimmung der Vertikalposition des Konkrements aus den Größen d, d' und z'.

9. Vorrichtung nach Anspruch 8, wobei der Abstand z des Konkrements (3) von der Strahlungsquelle (5) bestimmt wird als $z = z' \cdot \frac{d}{d'}$.

10. Vorrichtung nach Anspruch 8 oder 9, wobei die Strahlungsquelle eine Röntgenquelle ist.

11. Vorrichtung nach Anspruch 8, 9 oder 10, wobei die Therapievorrichtung eine Vorrichtung zur Stoßwellentherapie ist.

**12.** Vorrichtung nach einem der Ansprüche 8 bis 11, aufweisend einen horizontal in Körperlängsrichtung und/oder Körperquerrichtung beweglichen Behandlungstisch (9).

**13.** Vorrichtung nach einem der Ansprüche 8 bis 12, wobei die Bildgebungseinrichtung (4) horizontal in Körperlängsrichtung und/oder in Körperquerrichtung bewegbar ist.

**Fig. 1**

**Fig. 2**

**Fig. 3**

**Fig. 4**

Projektionsabbildung

Strahlungsquelle

Fig. 5

Steinlage (Objektebene)

Eingangsfenster (Bildebene)

Projektionsabbildung des Konkrements
an einer ersten Position

S1

Erfassung der Bildkoordinaten des
Konkrements in der ersten Position

S2

Horizontales Verfahren des Körpers relativ
zur Strahlungsquelle um Verfahrweg d

S3

Erfassung der Bildkoordinaten des
Konkrements in der zweiten Position

S4

Bestimmung der Projektion d' des
Verfahrwegs als Differenz der
Bildkoordinaten

S5

Bestimmung des vertikalen Abstandes z des
Konkrements von der Strahlungsquelle als
z=z' · d/d', wobei z' der Abstand der
Strahlungsquelle von der Bildebene ist

S6

**Fig. 6**

**Europäisches Patentamt**

# EUROPÄISCHER RECHERCHENBERICHT

Nummer der Anmeldung

EP 02 02 4041

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 2 329 187 A (DAY ROY C ET AL) 14. September 1943 (1943-09-14) * Seite 1, Spalte 2, Zeile 16 - Zeile 22 * * Seite 2, Spalte 2, Zeile 8 - Seite 3, Spalte 1, Zeile 25; Abbildungen 1,5-8 * | 1-13 | A61B17/22 A61B6/12 |
| X | US 2 192 887 A (BLACK LAWRENCE F) 12. März 1940 (1940-03-12) * Seite 1, Spalte 2, Zeile 45 - Seite 2, Spalte 1, Zeile 31; Abbildungen 1,2,4 * | 1-13 | |
| X | DE 29 09 476 A (PHILIPS PATENTVERWALTUNG) 18. September 1980 (1980-09-18) * Anspruch 1; Abbildung 3 * | 1-13 | |
| X,D | DE 10 29 122 B (MARIANNE SCHMIDTBERGER DR MED;PETER DESERNO; BRUNO JACOBS; JOSEF DERIS) 30. April 1958 (1958-04-30) * Spalte 3, Zeile 27 - Spalte 4, Zeile 5; Abbildungen 1,2 * | 1-13 | |
| A,D | DE 195 13 400 A (DORNIER MEDIZINTECHNIK) 10. Oktober 1996 (1996-10-10) * Zusammenfassung; Abbildung 1 * | 1,8 | RECHERCHIERTE SACHGEBIETE (Int.Cl.7) A61B |

Der vorliegende Recherchenbericht wurde für alle Patentansprüche erstellt

| Recherchenort | Abschlußdatum der Recherche | Prüfer |
|---|---|---|
| DEN HAAG | 12. März 2003 | Moers, R |

EPO FORM 1503 03 82 (P04C03)

**EP 1 317 907 A1**

**ANHANG ZUM EUROPÄISCHEN RECHERCHENBERICHT**
**ÜBER DIE EUROPÄISCHE PATENTANMELDUNG NR.**     EP 02 02 4041

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-03-2003

| Im Recherchenbericht angeführtes Patentdokument | | Datum der Veröffentlichung | Mitglied(er) der Patentfamilie | | Datum der Veröffentlichung |
|---|---|---|---|---|---|
| US 2329187 | A | 14-09-1943 | KEINE | | |
| US 2192887 | A | 12-03-1940 | KEINE | | |
| DE 2909476 | A | 18-09-1980 | DE | 2909476 A1 | 18-09-1980 |
| DE 1029122 | B | 30-04-1958 | KEINE | | |
| DE 19513400 | A | 10-10-1996 | DE | 19513400 A1 | 10-10-1996 |
| | | | EP | 0737439 A2 | 16-10-1996 |
| | | | JP | 8317931 A | 03-12-1996 |

EPO FORM P0461

Für nähere Einzelheiten zu diesem Anhang : siehe Amtsblatt des Europäischen Patentamts, Nr.12/82

11